# EUROPEAN PATENT APPLICATION

(11) **EP 4 778 549 A1**
(43) Date of publication of application: **22.07.2026**
(21) Application number: 26151703.1
(22) Date of filing: 14.01.2026
(51) Int. Cl.: A61M 5/145, A61M 5/172, A61M 5/315

(54) **DUAL-MOTOR SYRINGE DRIVER**

(30) Priority: 16.01.2025 US 202563745888 P
(71) Applicant: Fresenius Kabi USA, LLC, Lake Zurich, IL 60047 (US)
(72) Inventor: POWERS, Benjamin G., Lake Zurich, 60047 (US); WOLFF, Rémi, Lake Zurich, 60047 (US)
(74) Representative: Maikowski & Ninnemann Patentanwälte Partnerschaft mbB

(57) **Abstract**

Systems and methods are provided for driving a syringe pump. The syringe pump includes a syringe, a pusher movably received within a barrel of the syringe, and a drive assembly associated to the pusher and configured to be actuated to move the pusher through the barrel. The drive assembly includes a lead screw associated to the pusher, a planetary gearbox, a first motor associated to the lead screw via the planetary gearbox, and a second motor associated to the lead screw via the planetary gearbox. Actuation of the first motor and/or the second motor causes rotation of the lead screw so as to cause movement of the pusher through the barrel, with the direction and speed of the movement of the pusher through the barrel being dependent upon the direction of rotation and the rotational rate of the lead screw.

## Description

### Background

### Field of the Disclosure

The present subject matter relates to syringe pumps. More particularly, the present subject matter relates to the use of two motors to drive a syringe pump.

### Description of Related Art

It is known to employ syringe pumps in various settings to convey fluids, with such pumps typically being used when it is desirable to deliver small volumes of fluid at low, continuous flow rates with high precision. In one example, a syringe pump may be used to deliver a fluid (such as a medication) to a patient.

According to one conventional design, a syringe pump is driven by an assembly having a motor associated to a lead screw or nut that is associated to a pusher or plunger. The motor is actuated to rotate the lead screw, which causes the pusher to be moved through a barrel of a syringe. Operation of the motor in one direction (which may be referred to as the "reverse" direction) will cause the lead screw to rotate about its central axis to cause the pusher to be moved away from a tip of the syringe to draw fluid into the barrel, which may be referred to as "loading" the syringe. Operation of the motor in the opposite direction (which may be referred to as the "forward" direction) will cause the lead screw to rotate about its central axis in a direction that is opposite to its rotation when loading the syringe. Such rotation of the lead screw will cause the pusher to move through the barrel toward the tip, which forces fluid out of the syringe via the tip.

While it is typically advantageous for the pusher to be moved at a slow speed through the barrel, there are circumstances in which it is desirable for the pusher to be moved at a greater speed. For example, moving the pusher at a relatively high speed away from the tip of the syringe allows for the syringe to be loaded more quickly than when the pusher is moved at a lower speed. Additionally, the pusher must be moved at a relatively high speed toward the tip of the syringe in order to deliver a bolus of fluid from the syringe. It will thus be seen that the drive system of a syringe pump must be capable of producing precise movement of the pusher, both at relatively high speeds and at relatively low speeds. Furthermore, in some applications (e.g., drug delivery to a patient), it is important for the pusher to be driven at a low, continuous speed, such that a very small displacement resolution (on the order of less than one µm for one motor step) is advantageous, otherwise the fluid will be delivered via a series of micro-boluses instead of being delivered at a steady, continuous rate.

### Summary

There are several aspects of the present subject matter which may be embodied separately or together in the devices and methods described and claimed below. These aspects may be employed alone or in combination with other aspects of the subject matter described herein, and the description of these aspects together is not intended to preclude the use of these aspects separately or the claiming of such aspects separately as set forth in the claims appended hereto.

In one aspect, a syringe pump includes a syringe having a barrel and a tip, a pusher movably received within the barrel, and a drive assembly associated to the pusher and configured to be actuated to move the pusher through the barrel. The drive assembly includes a lead screw associated to the pusher, a planetary gearbox, a first motor associated to the lead screw via the planetary gearbox, and a second motor associated to the lead screw via the planetary gearbox. Actuation of the first motor and/or the second motor causes rotation of the lead screw so as to cause movement of the pusher through the barrel, with the direction and speed of the movement of the pusher through the barrel being dependent upon the direction of rotation and the rotational rate of the lead screw.

### Brief Description of the Drawings

Fig. 1 is a front perspective view of an exemplary syringe pump according to an aspect of the present disclosure;
Figs. 2 and 3 are rear perspective views of the syringe pump of Fig. 1, with Fig. 3 showing a cross-section of the syringe pump; and
Fig. 4 is a schematic view of the syringe pump of Fig. 1.

### Description of the Illustrated Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

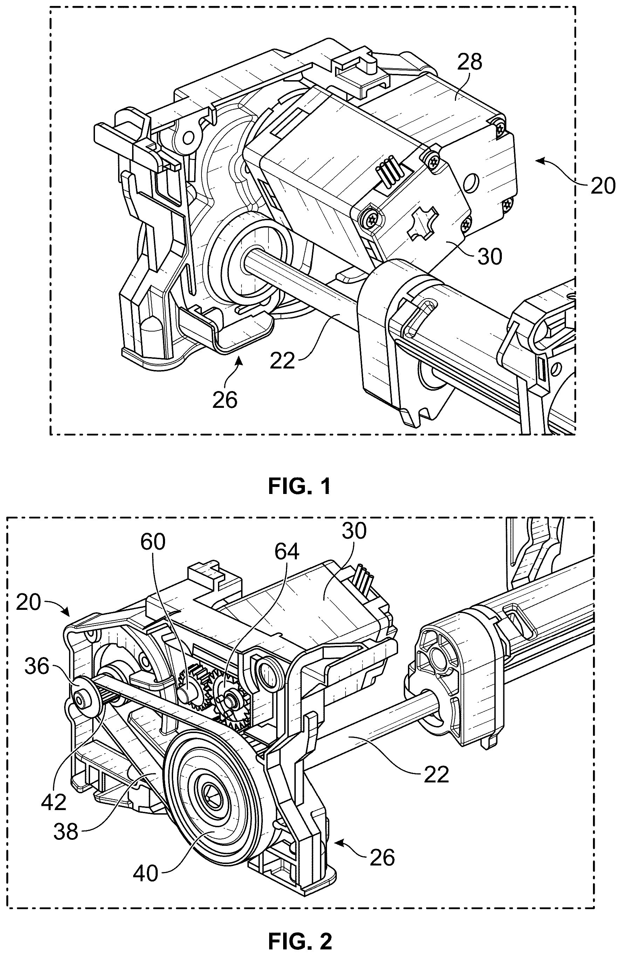

Figs. 1-4 show an exemplary embodiment of a syringe pump 10 embodying aspects of the present disclosure. The syringe pump 10 includes a syringe 12 defined by a barrel 14 and associated tip 16. A pusher 18 at least partially positioned within the barrel 14 is associated to a drive assembly 20 that is configured to be actuated to move the pusher 18 through the barrel 14. Movement of the pusher 18 away from the tip 16 will cause a substance to be drawn into the barrel 14 via the tip 16, while movement of the pusher 18 toward the tip 16 will cause a substance in the barrel 14 to be conveyed out of the syringe 12 via the tip 16. The nature of the substance may vary without departing from the scope of the present disclosure but, in one exemplary embodiment, the syringe pump 10 is configured as a medical device (or is incorporated into a larger medical device) for delivering a fluid, such as a medication, to a patient.

The drive assembly 20 includes a lead screw 22 that is rotatable about its central axis within a nut 24 and associated to the pusher 18 by any suitable mechanism that translates rotation of the lead screw 22 into linear movement of the pusher 18 through the barrel 14 of the syringe 12. The direction of rotation of the lead screw 22 determines the direction of movement of the pusher 18, with rotation of the lead screw 22 in one direction about its central axis causing the pusher 18 to move toward the tip 16 of the syringe 12 and rotation of the lead screw 22 in the opposite direction about its central axis causing the pusher 18 to move away from the tip 16. The rotational rate of the lead screw 22 determines the speed at which the pusher 18 moves through the barrel 14 of the syringe 12, with a greater rotational rate of the lead screw 22 resulting in faster movement of the pusher 18 than a lower rotational rate. In the illustrated embodiment the central axis of the lead screw 22 is substantially parallel to the direction of movement of the pusher 18 (with the pusher 18 being configured to move in a direction that coincides with the central axis of the lead screw 22 in other embodiments), but it should be understood that the pusher 18 may be configured to move in a direction that is substantially orthogonal to the central axis of the lead screw 22 or in a direction that is at some other angle with respect to the central axis of the lead screw 22.

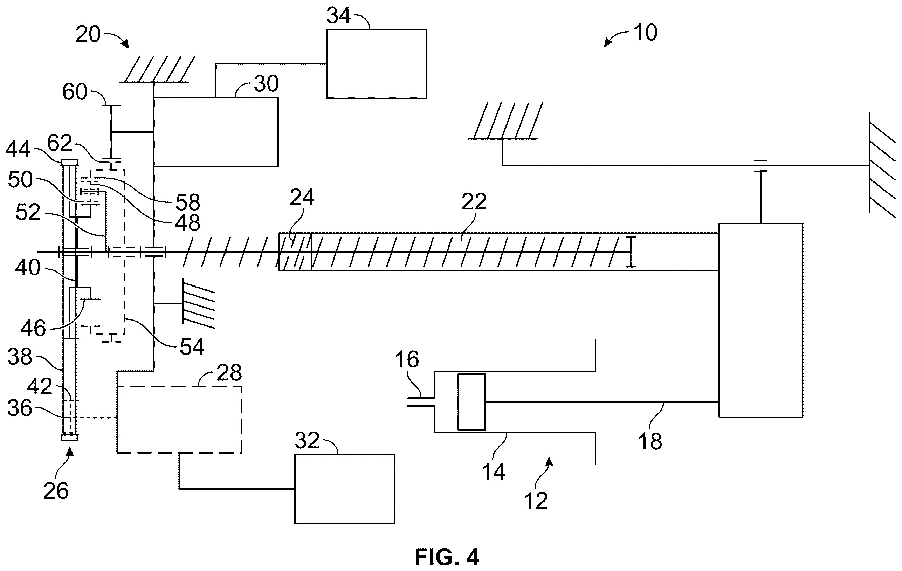

The drive assembly 20 also includes a planetary gearbox 26 (which will be described in greater detail herein), with a first or primary motor 28 and a second or secondary motor 30 each being associated to the lead screw 22 via the planetary gearbox 26. Each motor 28, 30 is associated to a suitable power source and controller (e.g., a microprocessor), with the power source energizing the motor 28, 30 and the controller controlling the operation of the motor 28, 30 so as to cause the desired movement of the pusher 18. Fig. 4 illustrates a first power source/controller 32 associated to the first motor 28 and a second power source/controller 34 associated to the second motor 30, though it should be understood that a single power source and/or a single controller may be associated to both of the motors 28 and 30.

The motors 28 and 30 may be variously configured without departing from the scope of the present disclosure, which may include the motors 28 and 30 being substantially identical or differently configured. In one embodiment, one or both of the motors 28 and 30 is configured as a stepper motor (with Figs. 1-3 showing both motors 28 and 30 configured as stepper motors). In another embodiment, one or both of the motors 28 and 30 is configured as a DC motor, which may be either a brushed DC motor or a brushless DC motor. A motor 28, 30 configured as a brushless DC motor may include sensors or may be sensor-less.

As for the planetary gearbox 26, an exemplary embodiment is shown in Figs. 1-4. In the illustrated embodiment, an output gear 36 is directly driven by the first motor 28. A drive belt 38 is wrapped around the output gear 36 and an input gear 40, such that rotation of the output gear 36 is transferred to the input gear 40 via the drive belt 38. The interface between the output gear 36 and the drive belt 38 is identified at 42, while the interface between the input gear 40 and the drive belt 38 is identified at 44. While the illustrated embodiment employs a drive belt 38 to transmit rotation of the output gear 36 to the input gear 40, it should be understood that rotation of the output gear 36 may be transmitted to the input gear 40 in other ways (including the output gear 36 being directly coupled to the input gear 40 via mating teeth or the output gear 36 being coupled to the input gear 40 via one or more intermediate gears) without departing from the scope of the present disclosure.

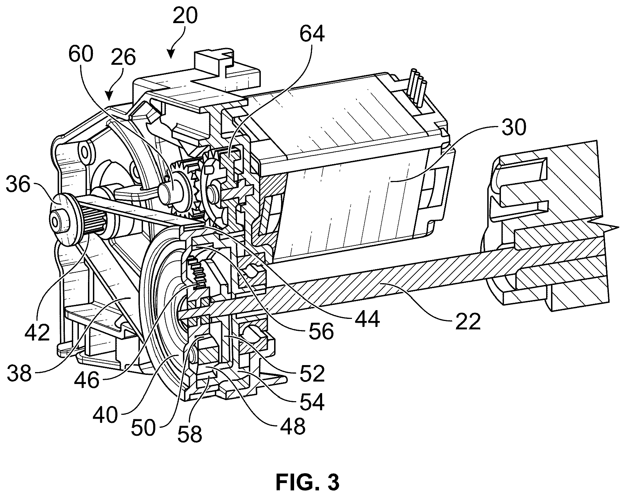

In addition to a first set of teeth defined along its outer perimeter (which interacts with the drive belt 38), the input gear 40 also includes a second set of teeth 46 positioned closer to its central axis (Fig. 3). As will be explained in greater detail, the second set of teeth 46 acts as the sun gear of the planetary portion of the gearbox 26, with one or more planet gears 48 mating with the input gear 40 at the second set of teeth 46. The interface between one of the planet gears 48 and the second set of teeth 46 of the input gear 40 is identified at 50.

The planet gears 48 ride on a carrier 52 that is rotationally coupled (i.e., fixed) to the lead screw 22, such that rotation of the carrier 52 is imparted to the lead screw 22. The planet gears 48 are also mated or meshed to a ring gear 54, with the interface between the teeth of one of the planet gears 48 and internal teeth 56 of the ring gear 54 being identified at 58. Rotational movement created by the second motor 30 is transmitted to the ring gear 54 via a pinion gear 60 (which is directly driven by the second motor 30), with the interface between teeth of the ring gear 54 and teeth of the pinion gear 60 being identified at 62 in Fig. 4. Figs. 2 and 3 show an idler gear 64 that associates the pinion gear 60 to the ring gear 54, though it should be understood that the idler gear 64 is included in the illustrated embodiment due to space considerations and does not impact the operation of the planetary gearbox 26. Further, while Figs. 2 and 3 show rotation of the pinion gear 60 being transmitted to the ring gear 54 via an idler gear 64 and Fig. 4 shows direct contact between the pinion gear 60 and the ring gear 54, it should be understood that rotation of the pinion gear 60 may be transmitted to the ring gear 54 in other ways (e.g., via a belt) without departing from the scope of the present disclosure.

It should also be understood that the planetary gearbox configuration shown in Figs. 1-4 and described above is merely exemplary and that the planetary gearbox 26 may be variously configured without departing from the scope of the present disclosure, provided that it is suitable for controlling movement of the pusher of a syringe pump according to the principles described herein. For example, while the motors 28 and 30 of the syringe pump 10 of Figs. 1-4 have different gear reductions to the lead screw 22 (which may be advantageous in order to optimize the controllable range for linear speed of the pusher 18 depending on the desired operational rates of the motors 28 and 30 and the resolution of speed control of the motor driver(s)), the first motor 28 and the second motor 30 may have the same gear reduction to the lead screw 22 in other embodiments. Further, while the illustrated embodiment employs stepper motors, differently configured motors (such as DC motors) may be used, as described above. Additional components (such as force sensors) may also be incorporated into the syringe pump without departing from the scope of the present disclosure.

Due to the configuration of the planetary gearbox 26, operation of the first motor 28 will rotate the output gear 36, with rotation of the output gear 36 being transmitted to the input gear 40 via the drive belt 38 (as described above). As the input gear 40 rotates, the planet gears 48 are driven by the sun gear portion of the input gear 40 (i.e., the second set of teeth 46), with the planet gears 48 rotating the carrier 52 and rotating against the ring gear 54. When the second motor 30 is idle, the ring gear 54 will be stationary, such that the carrier 52 and, thus, the lead screw 22 will rotate at a rate corresponding to the operational rate of the first motor 28. As the lead screw 22 rotates within the nut 24, the pusher 18 will move through the barrel 14 of the syringe 12, with the direction of movement of the pusher 18 being dependent upon the direction of operation of the first motor 28 and the speed of the movement of the pusher 18 being dependent upon the operational rate of the first motor 28.

On the other hand, when the first motor 28 is idle, the operation of the second motor 30 will rotate the pinion gear 60, which rotates the ring gear 54 (either directly or via the idler gear 64). The internal teeth 56 of the ring gear 54 transmit the rotation of the ring gear 54 to the planet gears 48, which rotate the carrier 52 and rotate against the sun gear portion (the second set of teeth 46) of the input gear 40. When the first motor 28 is idle, the input gear 40 (and, thus, its sun gear portion) will be stationary, such that the carrier 52 and the lead screw 22 will rotate at a rate corresponding to the operational rate of the second motor 30 and cause movement of the pusher 18 through the barrel 14 of the syringe 12. In this example, the direction of movement of the pusher 18 is dependent upon the direction of operation of the second motor 30 and the speed of the movement of the pusher 18 is dependent upon the operational rate of the second motor 30.

It will, thus, be seen that either motor 28, 30 can be used to drive motion of the pusher 18. Each motor 28, 30 is configured to be driven in a forward direction and in a reverse direction, with the associated controller determining the direction of operation of the motor 28, 30 and the operational rate. The motors 28 and 30 are associated to the lead screw 22 via the planetary gearbox 26 such that operation of either motor 28, 30 in the forward direction (while the other motor 28, 30 is idle) will cause the lead screw 22 to rotate in a direction that causes movement of the pusher 18 toward the tip 16 of the syringe 12. Similarly, the planetary gearbox 26 is configured such that operation of either motor 28, 30 in the reverse direction (while the other motor 28, 30 is idle) will cause the lead screw 22 to rotate in a direction that causes movement of the pusher 18 away from the tip 16 of the syringe 12.

Due to the configuration of the planetary gearbox 26, operation of both motors 28 and 30 in the same direction (with both operating in either the forward direction or in the reverse direction) will cause the lead screw 22 to rotate at a rotational rate that corresponds to the sum of the operational rates of the motors 28 and 30. More particularly, in the illustrated, operation of the first motor 28 will rotate the output gear 36, with rotation of the output gear 36 being transmitted to the input gear 40 via the drive belt 38 (as described above). As the input gear 40 rotates, the planet gears 48 are driven by the sun gear portion of the input gear 40 (i.e., the second set of teeth 46), as also described above. As further described above, operation of the second motor 30 will rotate the pinion gear 60, which rotates the ring gear 54, with the internal teeth 56 of the ring gear 54 transmitting the rotation of the ring gear 54 to the planet gears 48. When both motors 28 and 30 are operating in the same direction, the sun gear portion of the input gear 40 will rotate the planet gears 48 in the same direction in which the ring gear 54 is rotating, effectively causing the carrier 52 (and, thus, the lead screw 22) to rotate at a rate that corresponds to the sum of the operational rates of the motors 28 and 30. Thus, operating both motors 28 and 30 in the forward direction allows for especially fast movement of the pusher 18 toward the tip 16 of the syringe 12, while operating both motors 28 and 30 in the reverse direction allows for especially fast movement of the pusher 18 away from the tip 16 of the syringe 12.

On the other hand, when the motors 28 and 30 are operated in opposite directions (with one being operated in the forward direction and the other being operated in the reverse direction), the sun gear portion of the input gear 40 will rotate the planet gears 48 in a direction that is opposite to the direction in which the ring gear 54 is rotating, effectively causing the carrier 52 (and, thus, the lead screw 22) to rotate at a rate that corresponds to the difference between the effective operational rates of the motors 28 and 30. As used herein, the term "effective operational rate" refers to the rotational rate that would be imparted to the lead screw 22 by operation of a motor 28, 30, which is affected by the operational rate of the motor 28, 30 (e.g., the rpm at which the motor is operating) and the gear reduction to the lead screw 22. Thus, two motors functioning at the same "operational rate" or rpm may have a different "effective operational rate" if they have different gear reductions. Similarly, it is possible for two motors operating at different rpm or "operational rates" to be operating at the same "effective operational rate" if they have different gear reductions.

When the motor 28, 30 being operated in the forward direction is acting at a greater effective operational rate than the motor 28, 30 being operated in the reverse direction, the lead screw 22 will be rotated in a direction that causes the pusher 18 to move toward the tip 16 of the syringe 12. On the other hand, when the motor 28, 30 being operated in the reverse direction is acting at a greater effective operational rate than the motor 28, 30 being operated in the forward direction, the lead screw 22 will be rotated in a direction that causes the pusher 18 to move away from the tip 16 of the barrel 14. In the event that the motors 28 and 30 are operated in opposite directions at the same effective operational rate (such that the difference between the effective operational rates of the motors 28 and 30 is zero), there will be no rotation of carrier 52 or the lead screw 22 (due to the rotational rates of the sun gear portion of the input gear 40 and of the ring gear 54 effectively canceling each other out) and, hence, no movement of the pusher 18.

Turning now to the consequences and advantages of a syringe pump 10 that is configured according to aspects of the present disclosure, such a pump will be capable of more rapid loading and unloading of a syringe, on account of two motors being employed (rather than the single motor of a conventional syringe pump), with the total output speed corresponding to the sum of the operational rates (or effective operational rates) of the motors. Thus, if the motors 28 and 30 of a syringe pump 10 according to the present disclosure were to be identical to the single motor of a conventional system (with all three motors having the same gear reduction), the syringe pump 10 of the present disclosure would be capable of operating at twice the speed of the conventional pump. Furthermore, reconfiguring a conventional pump for increased speed (e.g., by providing a high-pitch lead screw and a low-ratio gearbox) would not be compatible with the small displacement resolution that is desirable for precise delivery at low rates, such that syringe pumps according to the present disclosure are capable of operating at speeds that are not practicable for conventional systems.

In addition to being capable of operating at high speeds for fast syringe loading and rapid bolus delivery, syringe pumps according to the present disclosure also have excellent (almost infinite) displacement resolution, on account of the velocity differential function of the planetary gearbox 26. For example, it may be desired for the pusher of a syringe pump to be moved at a very slow speed, such as on the order of 1 µm in five minutes. In such an example, if the resolution of a conventional syringe pump having a stepper motor were 0.2 µm, then the motor would be required to provide one pulse every sixty seconds, which may be considered as a succession of micro-boluses, instead of a continuous infusion. On the other hand, if a syringe pump according to the present disclosure were to have two stepper motors (with identical gear reductions) and the same resolution, the first motor 28 could be operated at sixty forward or positive steps and the second motor 30 could be operated at fifty-nine reverse or negative steps per sixty seconds, which would result in the same advance of one global step, but with sixty increments instead of one, effectively increasing the device resolution by sixty. By such an approach, in addition to providing excellent displacement resolution, this arrangement also provides for very steady and accurate movement of the pusher 18 at low rates, with a substance being continuously conveyed out of the syringe 12 at a uniform rate, rather than being delivered via a series of micro-boluses. As such, syringe pumps according to the present disclosure are well-suited for applications requiring the controlled delivery of substances at very low, uniform flow rates, such as delivering certain medical fluids to a patient.

In the preceding example, stepper motors (which move a fixed angular distance for each step or micro-step commanded) are employed, but syringe pumps according to the present disclosure may provide additional benefits when brushless DC motors are used. Brushless DC motors may be desirable because they have a very long life compared to stepper motors, with sensor-less brushless DC motors being further advantageous on account of being relatively inexpensive and having fewer components that could fail compared to DC motors employing Hall-effect sensors. However, sensor-less brushless DC motors rely on sensing the back electromotive force ("EMF") through the motor coils to control the electrical commutation of the motor, with it being necessary for the motor to be operating at a sufficiently high speed (e.g., on the order of 3,000 rpm) for the back EMF sensing to work properly. As such, sensor-less brushless DC motors are typically not viable for syringe pump applications, where the linear velocity of the pusher (and, thus, the rotational rate of the lead screw and the operational rate of the motor) needs to be very low. However, the velocity differential function of the planetary gearbox of a syringe pump according to the present disclosure allows for the use of sensor-less brushless DC motors by controlling its motors 28 and 30 to operate in opposing directions at operational rates that are nominally different. For example, the first motor 28 could be operated in the forward direction at an effective operational rate of 4,001 rpm and the second motor 30 could be operated in the reverse direction at an effective operational rate of 4,000 rpm, resulting in a total output speed of 1 rpm. By such an approach, a very low pusher velocity may be achieved while operating the motors 28 and 30 at high enough speeds for the back EMF sensing to work properly.

### Aspects

Aspect 1. A syringe pump comprising: a syringe including a barrel and a tip; a pusher movably received within the barrel; and a drive assembly associated to the pusher and configured to be actuated to move the pusher through the barrel, wherein the drive assembly includes a lead screw associated to the pusher, a planetary gearbox, a first motor associated to the lead screw via the planetary gearbox, and a second motor associated to the lead screw via the planetary gearbox, actuation of the first motor and/or the second motor causes rotation of the lead screw so as to cause movement of the pusher through the barrel, and a direction and a speed of the movement of the pusher through the barrel are dependent upon a direction of rotation and a rotational rate of the lead screw.

Aspect 2. The syringe pump of Aspect 1, wherein the planetary gearbox is configured such that actuation of the first motor in a forward direction and actuation of the second motor in the forward direction causes movement of the pusher toward the tip of the syringe.

Aspect 3. The syringe pump of Aspect 2, wherein the planetary gearbox is configured such that actuation of the first motor in the forward direction at a first rate and actuation of the second motor in the forward direction at a second rate causes the rotational rate of the lead screw to correspond to a sum of the first rate and the second rate.

Aspect 4. The syringe pump of any one of the preceding Aspects, wherein the planetary gearbox is configured such that actuation of the first motor in a reverse direction and actuation of the second motor in the reverse direction causes movement of the pusher away from the tip of the syringe.

Aspect 5. The syringe pump of Aspect 4, wherein the planetary gearbox is configured such that actuation of the first motor in the reverse direction at a first rate and actuation of the second motor in the reverse direction at a second rate causes the rotational rate of the lead screw to correspond to a sum of the first rate and the second rate.

Aspect 6. The syringe pump of any one of the preceding Aspects, wherein the planetary gearbox is configured such that actuation of the first motor in a forward direction and actuation of the second motor in a reverse direction causes movement of the pusher toward the tip of the syringe when an effective operational rate of the first motor is greater than an effective operational rate of the second motor.

Aspect 7. The syringe pump of Aspect 6, wherein the planetary gearbox is configured such that actuation of the first motor in the forward direction at a first effective operational rate and actuation of the second motor in the reverse direction at a second effective operational rate causes the rotational rate of the lead screw to correspond to a difference between the first effective operational rate and the second effective operational rate.

Aspect 8. The syringe pump of any one of the preceding Aspects, wherein the planetary gearbox is configured such that actuation of the first motor in a reverse direction and actuation of the second motor in a forward direction causes movement of the pusher away from the tip of the syringe when an effective operational rate of the first motor is greater than an effective operational rate of the second motor.

Aspect 9. The syringe pump of Aspect 8, wherein the planetary gearbox is configured such that actuation of the first motor in the reverse direction at a first effective operational rate and actuation of the second motor in the forward direction at a second effective operational rate causes the rotational rate of the lead screw to correspond to a difference between the first effective operational rate and the second effective operational rate.

Aspect 10. The syringe pump of any one of the preceding Aspects, wherein the planetary gearbox is configured such that actuation of the first motor in a forward direction and actuation of the second motor in a reverse direction causes no movement of the pusher when effective operational rates of the first motor and the second motor are the same.

Aspect 11. The syringe pump of any one of the preceding Aspects, wherein the planetary gearbox is configured such that actuation of the first motor in a reverse direction and actuation of the second motor in a forward direction causes no movement of the pusher when effective operational rates of the first motor and the second motor are the same.

Aspect 12. The syringe pump of any one of the preceding Aspects, wherein the planetary gearbox is configured such that actuation of the first motor in a forward direction while the second motor is idle causes movement of the pusher toward the tip of the syringe.

Aspect 13. The syringe pump of any one of the preceding Aspects, wherein the planetary gearbox is configured such that actuation of the first motor in a reverse direction while the second motor is idle causes movement of the pusher away from the tip of the syringe.

Aspect 14. The syringe pump of any one of the preceding Aspects, wherein the first motor and the second motor are configured as stepper motors.

Aspect 15. The syringe pump of any one of Aspects 1-13, wherein the first motor and the second motor are configured as brushed DC motors.

Aspect 16. The syringe pump of any one of Aspects 1-13, wherein the first motor and the second motor are configured as brushless DC motors.

Aspect 17. The syringe pump of Aspect 16, wherein the first motor and the second motor are configured as sensor-less, brushless DC motors.

Aspect 18. The syringe pump of any one of the preceding Aspects, wherein the planetary gearbox is configured such that the first motor and the second motor have the same gear reduction to the lead screw.

Aspect 19. The syringe pump of any one of Aspects 1-17, wherein the planetary gearbox is configured such that the first motor and the second motor have different gear reductions to the lead screw.

Aspect 20. The syringe pump of any one of the preceding Aspects, wherein the syringe pump is configured to deliver a fluid to a patient.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A syringe pump (10) comprising:
a syringe (12) including a barrel (14) and a tip (16);
a pusher (18) movably received within the barrel (14); and
a drive assembly (20) associated to the pusher (18) and configured to be actuated to move the pusher (18) through the barrel (14), wherein
the drive assembly (20) includes
a lead screw (22) associated to the pusher (18),
a planetary gearbox (26),
a first motor (28) associated to the lead screw (22) via the planetary gearbox (26), and
a second motor (30) associated to the lead screw (22) via the planetary gearbox (26),
actuation of the first motor (28) and/or the second motor (30) causes rotation of the lead screw (22) so as to cause movement of the pusher (18) through the barrel (14), and
a direction and a speed of the movement of the pusher (18) through the barrel (14) are dependent upon a direction of rotation and a rotational rate of the lead screw (22).

2. The syringe pump (10) of claim 1, wherein the planetary gearbox (26) is configured such that actuation of the first motor (28) in a forward direction and actuation of the second motor (30) in the forward direction causes movement of the pusher (18) toward the tip (16) of the syringe (12), with the planetary gearbox (26) preferably being configured such that actuation of the first motor (28) in the forward direction at a first rate and actuation of the second motor (30) in the forward direction at a second rate causes the rotational rate of the lead screw (22) to correspond to a sum of the first rate and the second rate.

3. The syringe pump (10) of any one of the preceding claims, wherein the planetary gearbox (26) is configured such that actuation of the first motor (28) in a reverse direction and actuation of the second motor (30) in the reverse direction causes movement of the pusher (18) away from the tip (16) of the syringe (12), with the planetary gearbox (26) preferably being configured such that actuation of the first motor (28) in the reverse direction at a first rate and actuation of the second motor (30) in the reverse direction at a second rate causes the rotational rate of the lead screw (22) to correspond to a sum of the first rate and the second rate.

4. The syringe pump (10) of any one of the preceding claims, wherein the planetary gearbox (26) is configured such that actuation of the first motor (28) in a forward direction and actuation of the second motor (30) in a reverse direction causes movement of the pusher (18) toward the tip (16) of the syringe (12) when an effective operational rate of the first motor (28) is greater than an effective operational rate of the second motor (30), with the planetary gearbox (26) preferably being configured such that actuation of the first motor (28) in the forward direction at a first effective operational rate and actuation of the second motor (30) in the reverse direction at a second effective operational rate causes the rotational rate of the lead screw (22) to correspond to a difference between the first effective operational rate and the second effective operational rate.

5. The syringe pump (10) of any one of the preceding claims, wherein the planetary gearbox (26) is configured such that actuation of the first motor (28) in a reverse direction and actuation of the second motor (30) in a forward direction causes movement of the pusher (18) away from the tip (16) of the syringe (12) when an effective operational rate of the first motor (28) is greater than an effective operational rate of the second motor (30), with the planetary gearbox (26) preferably being configured such that actuation of the first motor (28) in the reverse direction at a first effective operational rate and actuation of the second motor (30) in the forward direction at a second effective operational rate causes the rotational rate of the lead screw (22) to correspond to a difference between the first effective operational rate and the second effective operational rate.

6. The syringe pump (10) of any one of the preceding claims, wherein the planetary gearbox (26) is configured such that actuation of the first motor (28) in a forward direction and actuation of the second motor (30) in a reverse direction causes no movement of the pusher (18) when effective operational rates of the first motor (28) and the second motor (30) are the same.

7. The syringe pump (10) of any one of the preceding claims, wherein the planetary gearbox (26) is configured such that actuation of the first motor (28) in a reverse direction and actuation of the second motor (30) in a forward direction causes no movement of the pusher (18) when effective operational rates of the first motor (28) and the second motor (30) are the same.

8. The syringe pump (10) of any one of the preceding claims, wherein the planetary gearbox (26) is configured such that actuation of the first motor (28) in a forward direction while the second motor (30) is idle causes movement of the pusher (18) toward the tip (16) of the syringe (12).

9. The syringe pump (10) of any one of the preceding claims, wherein the planetary gearbox (26) is configured such that actuation of the first motor (28) in a reverse direction while the second motor (30) is idle causes movement of the pusher (18) away from the tip (16) of the syringe (12).

10. The syringe pump (10) of any one of the preceding claims, wherein the first motor (28) and the second motor (30) are configured as stepper motors.

11. The syringe pump (10) of any one of claims 1-9, wherein the first motor (28) and the second motor (30) are configured as brushed DC motors.

12. The syringe pump (10) of any one of claims 1-9, wherein the first motor (28) and the second motor (30) are configured as brushless DC motors, which are preferably sensor-less.

13. The syringe pump (10) of any one of the preceding claims, wherein the planetary gearbox (26) is configured such that the first motor (28) and the second motor (30) have the same gear reduction to the lead screw (22).

14. The syringe pump (10) of any one of claims 1-12, wherein the planetary gearbox (26) is configured such that the first motor (28) and the second motor (30) have different gear reductions to the lead screw (22).

15. The syringe pump (10) of any one of the preceding claims, wherein the syringe pump (10) is configured to deliver a fluid to a patient.
